(19) Europäisches Patentamt
European Patent Office
Office européen des brevets

(11) Veröffentlichungsnummer: **0 273 272 B1**

(12) **EUROPÄISCHE PATENTSCHRIFT**

(45) Veröffentlichungstag der Patentschrift: **06.05.92**

(51) Int. Cl.[5]: **A61K 31/445**

(21) Anmeldenummer: **87118367.9**

(22) Anmeldetag: **11.12.87**

# (54) 17-Benzylspartein-Derivate enthaltende positiv inotrop wirksame pharmazeutische Zubereitungen.

(30) Priorität: **19.12.86 DE 3643402**

(43) Veröffentlichungstag der Anmeldung:
**06.07.88 Patentblatt 88/27**

(45) Bekanntmachung des Hinweises auf die Patenterteilung:
**06.05.92 Patentblatt 92/19**

(84) Benannte Vertragsstaaten:
**AT BE CH DE FR GB IT LI NL**

(56) Entgegenhaltungen:
**EP-A- 0 206 155**

**E.F. REYNOLDS: "Martindale, The Extra Pharmacopoeia", 28. Edition, 1982, Seite 1756, Nr. 13264s, The Pharmaceutical Press, London, GB**

**CHEMICAL ABSTRACTS, Band 51, Nr. 11, 10. Juni 1957, Spalte 8114d, Columbus, Ohio, US; M. RINK et al.: "17-Hydroxysparteine", & ARCH. PHARM. 289, 695-702 (1956)**

(73) Patentinhaber: **Kali-Chemie Pharma GmbH**
**Hans-Böckler-Allee 20 Postfach 220**
**W-3000 Hannover 1(DE)**

(72) Erfinder: **Schön, Uwe**
**Föhrenkamp 12**
**W-3167 Burgdorf(DE)**
Erfinder: **Kehrbach, Wolfgang**
**Altenbekener Damm 41**
**W-3000 Hannover 1(DE)**
Erfinder: **Hachmeister, Bernd**
**Mövenkamp 4**
**W-3004 Isernhagen 1(DE)**
Erfinder: **Buschmann, Gerd**
**Ernst-Ebeling-Strasse 9**
**W-3000 Hannover 72(DE)**
Erfinder: **Kühl, Ulrich**
**Franzburger Strasse 10**
**W-3007 Gehrden 1(DE)**

(74) Vertreter: **Lauer, Dieter, Dr.**
**c/o Kali-Chemie Aktiengesellschaft Postfach 220**
**W-3000 Hannover 1(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Verwendung von in 17-Stellung einen gegebenenfalls substituierten Benzylrest tragenden Spartein-Derivaten zur Behandlung von ischämiebedingter und nichtischämiebedingter Herzinsuffizienz und zur Herstellung von für diese Behandlung geeigneten Arzneimitteln, welche als Wirkstoff eine positiv inotrop wirksame Menge der vorgenannten 17-Benzylspartein-Derivate enthalten.

Der Erfindung liegt die Aufgabe zugrunde, neue positiv inotrop wirksame pharmazeutische Zubereitungen mit verbessertem Wirkungsprofil zur Behandlung von Herzinsuffizienzen zu entwickeln.

Die bekanntesten Arzneimittel mit positiv inotroper Wirkung sind Herzglycoside (Digitalis- und Strophantin-Produkte). Diese führen neben ihrer das Schlagvolumen erhöhenden positiv inotropen Wirkung indirekt auch zu einer Abnahme der Herzfrequenz durch Anpassung an das erhöhte Schlagvolumen. Der große Nachteil der Herzglycoside liegt jedoch in ihrer außerordentlich geringen therapeutischen Breite, welche insbesondere durch die Herzarrhythmien auslösenden Eigenschaften dieser Präparate begrenzt ist.

Alle sonstigen auf dem Markt befindlichen positiv inotrop wirksamen Arzneimittel haben den Nachteil, daß sie die Herzfrequenz dabei nicht senken und in vielen Fällen sogar eine Herzfrequenzsteigerung bewirken. Dies führt zu einer gerade bei Herzinsuffizienz unerwünschten Mehrbelastung des Herzens.

Erfindungsgemäß werden zur Herstellung von zur Steigerung der Kontraktionskraft des Herzens bei Menschen und größeren Säugetieren geeigneten Arzneimitteln als positiv inotrop wirkende Wirkstoffe 17-Benzylspartein-Derivate der allgemeinen Formel I
(siehe Formel I)
worin die Reste $R^1$, $R^2$ und $R^3$ unabhängig voneinander Wasserstoff, Alkyl mit 1-4 Kohlenstoffatomen, Alkoxy mit 1-4 Kohlenstoffatomen, Halogen, Trifluormethyl oder Hydroxy bedeuten, sowie deren pharmakologisch akzeptable Säureadditionssalze verwendet.

Von den erfindungsgemäß verwendeten Wirkstoffen der Formel I ist das unsubstituierte 17-Benzylspartein bereits bekannt (Rink, et al, Archiv der Pharmazie 289, 1956, Seite 702). Über dessen pharmakologische Eigenschaften ist jedoch noch nichts publiziert.

Die erfindungsgemäß zur Behandlung von Herzinsuffizienz verwendeten Wirkstoffe der Formel I fallen ferner unter den Umfang von in unserer nicht vorveröffentlichten deutschen Anmeldung P 35 22 475.4 bzw. der entsprechenden ebenfalls nicht vorveröffentlichten europäischen Anmeldung EP-A-206155 beschriebenen in 17-Stellung einen aromatischen Substituenten tragenden Spartein-Derivaten mit herzfrequenzsenkenden und antiarrhythmischen Wirkungen.

Die Verbindungen können auch nach den in diesen noch nicht vorveröffentlichten Patentanmeldungen beschriebenen Verfahren hergestellt werden.

Es wurde nun überraschenderweise gefunden, daß die Verbindungen der Formel I sich durch ein neuartiges Wirkungsprofil am Herzen auszeichnen und neben den vorgenannten herzfrequenzsenkenden und antiarrhythmischen Eigenschaften auch ausgeprägte positiv inotrope Wirkungen besitzen.

Die Kombination von positiv inotroper, also die Kontraktionskraft des Herzens steigernder, Wirkung mit einer herzfrequenzsenkenden Wirkungskomponente führt zu einer Ökonomisierung der Herzarbeit. Trotz Zunahme der Pumpleistung erfolgt keine entsprechende Steigerung des Sauerstoffbedarfes des Herzens. Dies ist eine zur Behandlung von Herzinsuffizienz äußerst erwünschte Wirkkombination, welche bisher mit nicht zu den Herzglycosiden gehörenden positiv inotropen Arzneimitteln nicht erreicht werden konnte und einen wesentlichen Vorteil bei der Behandlung der Herzinsuffizienz darstellt.

Dabei zeichnen sich die erfindungsgemäßen Verbindungen ferner durch eine große therapeutische Breite aus und besitzen auch in hohen Dosen keine arrhythmogenen Nebenwirkungen.

In den Verbindungen der Formel I kommen als Alkylreste geradkettiges oder verzweigtes Alkyl mit 1 bis 4, vorzugsweise 1 bis 2 Kohlenstoffatomen, insbesondere Methyl, in Frage. Alkoxyreste können ebenfalls geradkettig oder verzweigt sein und enthalten 1 bis 4, insbesondere 1 bis 2 Kohlenstoffatome und stellen vorzugsweise Methoxy dar. Als Halogensubstituenten kommen Fluor, Chlor oder Brom in Frage. Als Beispiele von monosubstituierten Verbindungen, seien solche Verbindungen genannt, worin $R^2$ und $R^3$ Wasserstoff bedeuten und $R^1$ die folgenden Bedeutungen besitzt: 2-Methyl, 3-Methyl, 4-Methyl, 2-Methoxy, 3-Methoxy, 4-Methoxy, 3-Hydroxy, 2-Fluor, 3-Fluor, 4-Fluor, 2-Chlor, 3-Chlor, 4-Chlor, 2-Brom, 3-Brom, 4-Brom, 3-Trifluormethyl. Als Beispiele disubstituierter Verbindungen seinen Verbindungen genannt, worin $R^3$ Wasserstoff bedeutet und $R^1$ und $R^2$ die folgenden Bedeutungen besitzen: 2,4-Dimethyl, 2,3-Dimethoxy, 3,4-Dichlor, 2,6-Dichlor, 3,5-Dichlor, 2-Fluor-3-Methyl, 2,6-Difluor, 2-Fluor-6-Chlor. Als Beispiel einer trisubstituierten Verbindung sei die 3,4,5-Trimethoxy Verbindung genannt.

Als pharmakologisch verwendbare Säureadditionssalze der Verbindungen der Formel I eignen sich beispielsweise wasserlösliche und wasserunlösliche Salze von anorganischen oder organischen Säuren wie z.B. das Hydrochlorid, Hydrobromid, Hydrojodid, Phosphat, Nitrat, Sulfat, Perchlorat, Acetat, Citrat, Gluconat, Benzoat, Propionat, Butyrat, Salicylat, Sulfosalicylat, Maleinat, Laurat, Fumarat, Succinat, Oxalat, Tartrat, Stearat, Tosylat (p-Toluolsulfonat), 2-Hydroxy-3-naphtoat, 3-Hydroxy-2-naphtoat, Mesylat (Methansulfonat), Naphtalinsulfonat.

Die positiv inotrope Wirkung der Verbindungen der Formel I und die günstige Kombination von positiv inotroper Wirkung und die Herzfrequenz senkender Wirkung läßt sich in pharmakologischen Standardtests in vivo an Tieren, beispielsweise an Ratten, nachweisen. Der positiv inotrope Effekt läßt sich auch in vitro am isolierten Rattenherz zeigen. Die antiarrhythmische Wirkung der Substanzen läßt sich in vitro am linken Herzvorhof des Meerschweinchens nachweisen.

Beschreibung der pharmakologischen Versuchsmethoden.

1) Untersuchungen in vivo an der narkotisierten Ratte.

Die Wirkung der Substanzen auf Blutdruck und Herzfrequenz bei i.v.-Dauerinfusion in narkotisierten Ratten wird nach der Methode von Buschmann et al. (J. Cardiovascular Pharmacol. 2 777-781 (1980)) bestimmt.

Männliche Wistar-Ratten (330-370 g Körpergewicht) werden durch i.p.-Applikation von 1,25 g/kg Urethan narkotisiert und tracheotomiert. Nach einer Äquilibrierungsphase von 10 Minuten wird mit den Messungen begonnen. In einer Vorlaufphase von 5 Minuten werden die Ausgangswerte gemessen. Anschließend werden die Prüfsubstanzen gelöst in isotonischer Natriumschloridlösung (gegebenenfalls mit Zusatz eines Lösungsvermittlers) als Dauerinfusion i.v. appliziert, beginnend mit einer Dosis vom 0,01 µmol/kg/min. Die Dosis wird alle 10 Minuten ohne Erhöhung des Infusionsvolumens auf das 10-fache gesteigert. Eine Kontrollgruppe erhält nur wirkstofffreie Lösung appliziert.

Der Einfluß der Wirksubstanzen auf die Kontraktionskraft des Herzens wurde anhand ihrer Beeinflussung der maximalen Anstiegsgeschwindigkeit des arteriellen Blutdrucks narkotisierter Ratten untersucht. Eine Zunahme der maximalen Anstiegsgeschwindigkeit des arteriellen Blutdrucks ist ein Index für eine Zunahme der Kontraktionskraft des Herzmuskels und wird deshalb als Parameter zur Untersuchung des Kontraktionsverhaltens verwendet (siehe Chan et al., J. Pharmacol. Methods 18, 23-29, 1987). Die maximale Druckanstiegsgeschwindigkeit wird aus dem Blutdruck durch elektronische Ermittlung des Differentialquotienten $dP/dt_{max}$ abgeleitet. In der nachfolgenden Tabelle 1 wird als maximal wirksame Dosis diejenige Dosis angegeben, bei der die maximale Druckanstiegsgeschwindigkeit $dP/dt_{max}$ ihren höchsten Wert erreicht hat. Außerdem werden die bei dieser Dosis gemessenen Änderungen der Herzfrequenz und des mittleren arteriellen Blutdrucks angegeben. Die Änderungen werden jeweils als Nettoprozent-Werte angegeben, das sind die bei der jeweiligen Dosis gemessenen Änderungen in Prozent des jeweiligen Ausgangswertes abzüglich der in der Kontrollgruppe zum gleichen Zeitpunkt gemessenen Prozent Änderungen. Wie aus der Tabelle 1 ersichtlich, steigern die Wirksubstanzen das $dP/dt_{max}$ und senken gleichzeitig die Herzfrequenz bei fehlender oder nur geringer Beeinflussung des mittleren arteriellen Blutdrucks.

Die für die Testsubstanzen in der Tabelle 1 angegebenen Beispielsnummern beziehen sich auf die nachstehenden Herstellungsbeispiele (siehe insbesondere Tabelle 5).

Tabelle 1

| Beinflussung von maximaler Anstiegsgschwindigkeit des arteriellen Blutdruckes ($dP/dt_{max}$), Herzfrequenz (FRQ) und mittlerem arteriellen Blutdruck (Pm). | | | | |
|---|---|---|---|---|
| Test-Subst. Bsp.Nr. | Maximal wirksame Dosis µmol/kg iv | Netto % Anstieg $dP/dt_{max}$ | Netto % Änderung FRQ | Netto % Änderung Pm |
| 502 | 9,1 | 57 | - 44 | + 5 |
| 500 | 11 | 50 | - 41 | + 8 |
| 501 | 10 | 49 | - 38 | + 6 |
| 6 | 26 | 49 | - 35 | - 4 |
| 504 | 26 | 30 | - 27 | + 3 |
| 7 | 11 | 30 | - 31 | + 8 |
| 518 | 8,6 | 67 | - 39 | + 4 |
| 505 | 11 | 79 | - 57 | - 1 |
| 509 | 16 | 50 | - 42 | - 4 |
| 510 | 9,6 | 56 | - 29 | + 19 |
| 511 | 11 | 46 | - 43 | - 4 |

In dem gleichen Versuchsmodell wurde auch der Einfluß der Wirksubstanzen auf den myokardialen Sauerstoffverbrauch an narkotisierten Ratten untersucht und anhand der Methode von Neill (Neill, W.Z., H.H. Levine, R.J. Wagman, R. Gorlin, Circulation Research 12 (1963), 163) berechnet. Dazu wird das Produkt aus systolischem Blutdruck und Herzfrequenz (= $\overline{\text{D}}$oppelprodukt mm Hg x $min^{-1}$ x 0,01) berechnet. Wie aus der nachstehenden Tabelle 1A ersichtlich ist, reduzieren die Wirksubstanzen dieses Doppelprodukt und führen damit zu einer Sauerstoffeinsparung am Herzen.

Tabelle 1A

| Einfluß auf Herzfrequenz (FRQ), systolischen Blutdruck ($P_5$) und das Doppelprodukt (DP) narkotisierter Ratten | | | | | |
|---|---|---|---|---|---|
| Test-subst. Bsp.Nr. | Dosis [µmol/kg i.v.] | FRQ [1/min] | $P_5$ [mm Hg] | DP [mm Hg/ min x 100] | Änderung DP [Prozent] |
| 502 *) | Vorwerte | 368 | 123 | 453 | - |
| | 6,6 | 254 | 124 | 312 | -29 |
| 505 *) | Vorwerte | 408 | 124 | 506 | - |
| | 2,6 | 306 | 121 | 376 | -26 |

*) = eingesetzt als Dihydrochlorid

2. Bestimmung der minimalen toxischen Dosis.

Männlichen Mäusen von 20-25 g Gewicht werden p.o. Maximaldosen von 300 mg/kg der Testsubstanz verabreicht. Die Tiere werden 3 Stunden lang sorgfältig auf Toxizitätssymptome beobachtet. über einen Zeitraum von 24 Stunden nach der Applikation werden zusätzlich alle Symptome und Todesfälle registriert. Begleitsymptome werden ebenfalls beobachtet und registriert. Wenn Tod oder starke toxische Symptome beobachtet werden, werden weiteren Mäusen zunehmend geringere Dosen verabreicht, bis keine toxischen Symptome mehr auftreten. Die niedrigste Dosis, welche Tod oder starke toxische Symptome hervorruft, wird als minimale toxische Dosis in der nachfolgenden Tabelle 2 angegeben.

Tabelle 2

| Substanz Beispiel Nr. | Minimale toxische Dosis mg/kg p.o. |
|---|---|
| 6 | >300 |
| 501 | 300 |
| 502 * | 300 |
| 505 * | 300 |
| 511 ** | >300 |
| 518 ** | >300 |
| 519 ** | 300 |
| 523 | 300 |
| 500 ** | >300 |
| 509 | >300 |

* als Dihydrochlorid eingesetzt
** als weinsaures Salz eingesetzt

3. In vitro Nachweis der antiarrhythmischen Wirkung.

Der Nachweis der antiarrhythmischen Wirkung der Wirksubstanzen erfolgte experimentell durch Bestimmung der funktionellen Refraktärzeit des linken Herzvorhofes von weiblichen Albino Pirbright-white Meerschweinchen der Gewichtsklasse 300 bis 400 g mit Hilfe der gepaarten elektrischen Stimulation in Anlehnung an die Methode von Govier (W.C. Govier, J. Pharmacol. Exp. Ther. 148 (1) (1965), 100 bis 105). In der nachfolgenden Tabelle 3 ist als FRP 125 % diejenige Konzentration in µmol/l angegeben, bei der es 18 min nach Substanzapplikation zu einer Verlängerung der funktionellen Refraktärzeit auf 125 % kommt.

Der direkte Einfluß der Wirksubstanz auf die Herzfrequenz (FRQ) wurde an spontan schlagenden, isolierten rechten Vorhöfen von weiblichen Albino Pirbright-white Meerschweinchen der Gewichtsklasse von 300 bis 400 g geprüft. In Tabelle 3 ist als FRQ 75 % diejenige Konzentration in µmol/l angegeben, bei der es 20 min nach der Substanzgabe zu einer Abnahme der Frequenz auf 75 % des Ausgangwertes kommt.

Tabelle 3

| Testsubstanz der Formel I Substanz Nr. * | Herzwirksame Eigenschaften Effektive Konzentration in µmol/l zur Erreichung von | |
|---|---|---|
| | FRQ 75 % | FRP 125 % |
| 500 *** | 1,46 | 3,52 |
| 501 | 4,69 | 3,71 |
| 502 ** | 2,06 | 6,24 |
| 505 ** | 0,99 | 2,17 |
| 509 | 4,09 | 2,83 |
| 511 | 5,12 | 2,47 |
| 518 | 3,13 | 2,58 |
| 519 | 1,9 | 3,24 |
| 523 | 0,95 | 2,69 |
| 6 | 4,85 | 3,39 |

* Die Substanzen werden in der in den Beispielstabellen angegebenen Form (Salz oder Base) verwendet, sofern nicht anders angegeben.
** eingesetzt als Dihydrochlorid
*** eingesetzt als weinsaures Salz

In diesem Versuchsmodell zeigt sich, daß im Dosisbereich der herzfrequenzsenkenden Wirkungen auch die Refraktarzeit verlängernde und somit antiarrhythmisch wirksame Eigenschaften auftreten.

Aus den Testergebnissen geht klar hervor, daß die Verbindungen der Formel I eine ausgeprägte positiv inotrope Wirkung in Kombination mit einer herzfrequenzsenkenden Wirkung besitzen und somit zur Behandlung von Herzinsuffizienzen besonders geeignet sind.

Als Wirkstoffe zur Behandlung von Herzinsuffizienzen und zur Herstellung von positiv inotrop wirksamen pharmazeutischen Zubereitungen können die Verbindungen der Formel I in Form der freien Basen oder ihrer physiologisch verträglichen Säureadditionssalze eingesetzt werden. Die zu verwendenden Dosen können individuell verschieden sein und variieren naturgemäß je nach Art des zu behandelnden Zustandes, der verwendeten Substanz und der Applikationsform. Z.B. werden parenterale Formulierungen im allgemeinen weniger Wirkstoff enthalten als orale Präparate. Im allgemeinen eignen sich zur Applikation am Menschen und an größeren Säugetieren jedoch Arzneiformen mit einem Wirkstoffgehalt von 0,5 bis 50, insbesondere 1 bis 20 mg Wirkstoff pro Einzeldosis.

Die Verbindungen können erfindunsgemäß zusammen mit üblichen pharmazeutischen Hilfs- und/oder Trägerstoffen in festen oder flüssigen pharmazeutischen Zubereitungen enthalten sein. Als Beispiele fester Präparate seien oral applizierbare Präparate wie Tabletten, Kapseln, Pulver, Granulate oder Dragees genannt oder auch Suppositorien. Fest Präparate können pharmazeutisch übliche anorganische und/oder organische Trägerstoffe wie z.B. Talkum, Milchzucker oder Stärke neben pharmazeutisch üblichen Hilfsmitteln, beispielsweise Gleitmitteln oder Tablettensprengmitteln enthalten. Flüssige Präparate wie Lösungen, Suspensionen oder Emulsionen können die üblichen Verdünnungsmittel wie Wasser, Öle und/oder Suspensionsmittel wie Polyäthylenglykole und dergleichen enthalten. Es können zusätzlich weitere Hilfsstoffe zugegeben werden, wie z.B. Konservierungsmittel, Geschmackskorrigenzien und dergleichen.

Die Wirkstoffe können mit den pharmazeutischen Hilfs- und/oder Trägerstoffen in an sich bekannter Weise gemischt und formuliert werden. Zur Herstellung fester Arzneiformen können die Wirkstoffe beispielsweise mit den Hilfs- und/ oder Trägerstoffen in üblicher Weise gemischt und naß oder trocken granuliert werden. Je nach Art der verwendeten Zusatzstoffe kann gegebenenfalls auch durch einfaches Mischen ein direkt tablettierbares Pulver erhalten werden. Das Granulat oder Pulver kann direkt in Kapseln abgefüllt oder in üblicher Weise zu Tablettenkernen verpreßt werden. Diese können gewünschtenfalls in bekannter Weise dragiert werden.

Die Verbindungen der Formel I können auf an sich bekannte Weise ausgehend von 17-Hydroxyspartein oder einem 17-Dehydrospartein-Salz, z.B. nach den in der nicht vorveröffentlichten europäischen Patentanmeldung EP-A-206155 beschriebenen Verfahren, hergestellt werden. Nach einer Verfahrensvariante i) wird dabei 17-Hydroxyspartein oder ein 17-Dehydrospartein-Salz, insbesondere das Perchlorat, mit einer Grignard-Verbindung der Formel (II)
(siehe Formel II)
worin $R^{1'}$, $R^{2'}$ und $R^{3'}$ die für $R^1$, $R^2$ und $R^3$ angegebene Bedeutung mit Ausnahme von Hydroxy haben und Hal Halogen, insbesondere Brom oder Chlor bedeutet, umgesetzt zu den entsprechenden Verbindungen der Formel (I).

In einer Variante ii) wird 17-Dehydrospartein-Salz, insbesondere das Perchlorat, mit einer metallorganischen Verbindung der Formel (III)
(siehe Formel III)
worin $R^{1'}$, $R^{2'}$ und $R^{3'}$ obige Bedeutung haben, umgesetzt zu den entsprechenden Verbindungen der Formel (I).

Die Herstellung der Verbindungen (II) bzw. (III) ist an sich bekannt und erfolgt durch Umsetzung von Benzylhalogeniden der Formel (IV)
(siehe Formel IV)
worin $R^{1'}$, $R^{2'}$, $R^{3'}$ und Hal obige Bedeutung besitzen, mit Magnesium, Lithium bzw. mit Alkyllithium, vorzugsweise n-Butyl-lithium.

In Verbindungen, in denen $R^{1'}$, $R^{2'}$ und/oder $R^{3'}$ Methoxy bedeuten, können in an sich bekannter Weise mit HJ die Methoxygruppe(n) zu Hydroxy gespalten werden.

Die nach den vorstehenden Verfahren gebildeten Verbindungen können als solche oder in Form ihrer pharmakologisch verwendbaren Säureadditionssalze gewonnen werden.

Die metallorganischen Reaktionen werden unter Ausschluß von Feuchtigkeit (absolutierte Lösungsmittel) und Luftsauerstoff (unter Inertgasatmosphäre, z.B. Stickstoff, Argon) durchgeführt.

Als inerte organische Lösungsmittel kommen in Frage Ether (z.B. Diethylether, Tetrahydrofuran, Dioxan, Dimethoxyethan) oder Kohlenwasserstoffe wie z.B. Hexan, Cyclohexan oder Benzol, vorzugsweise Diethylether, Tetrahydrofuran und Hexan bzw. deren Gemische.

In der Variante (i) erzeugt man zunächst aus dem entsprechenden Halogenid (IV) und fein verteiltem Magnesium entsprechend den Bedingungen der Grignard-Reaktion das Grignard-Reagenz (II) (siehe z.B. K. Nützel, Methoden zur Herstellung und Umwandlung magnesiumorganischer Verbindungen, in: Houben-Weyl, Methoden der organischen Chemie, Band 13/2a, S. 53 ff).

Zur Erzeugung des Grignard-Reagenzes kann, falls notwendig, ein Katalysator verwendet werden, z.B. Jod oder 1,2-Dibromethan. Die Reaktionstemperatur liegt üblicherweise zwischen Raumtemperatur und dem Siedepunkt des Lösungsmittels oder des Lösungsmittelgemisches.

In Abhängigkeit von sterischen und elektronischen Eigenschaften der Halogenide (IV) kann die Reaktionsdauer zwischen 30 min und mehreren Stunden betragen.

Alternativ kann gemäß der Variante ii) auch eine lithiumorganische Reaktion zur Erzeugung der Verbindung (I) durchgeführt werden. Die Darstellung der Lithium-Zwischenstufe erfolgt nach Standardmethoden (siehe z.B. H. Gilman, The Metalation Reaction With Organolithium Compounds, in: Organic Reactions, Vol. 8, S,. 258 ff oder H. R. Rodriguez, Heteroatom-Facilitated Lithiations, in: Organic Reactions, Vol. 26, S. 1 ff), beispielsweise Umsetzungen von Benzylhalogeniden (IV) oder entsprechend substituierten Toluolen mit Lithiumalkylen wie vorzugsweise n-Butyllithium oder Lithiumamiden wie vorzugsweise Lithiumdiisopropylamid.

Es ist bekannt, daß stickstoffhaltige Lewis-Basen die Reaktivität der Lithiumverbindungen steigern; daher werden, falls erforderlich, beispielsweise N,N,N',N'-Tetramethylethylendiamin (TMEDA) oder 1,4-Diazabicyclo-[2,2,2]-octan (DABCO) als Katalysatoren eingesetzt.

Die Reaktionstemperatur liegt üblicherweise zwischen -78 °C und $^+$40 °C. In Abhängigkeit von den sterischen und elektronischen Eigenschaften des Halogenids (IV) kann die Reaktionsdauer zwischen 30 min und mehreren Stunden betragen.

Die Etherspaltung erfolgt nach an sich üblichen Methoden wie Behandlung mit Jodwasserstoffsäure, Bortribromid, Bortrichlorid oder Phosphorpentachlorid, vorzugsweise mit Jodwasserstoffsäure.

Die Umsetzungen können bei Normaldruck oder auch bei erhöhtem Druck durchgeführt werden, vorzugsweise bei Normaldruck.

Die Reaktionstemperaturen können je nach Verfahren variieren in einem Bereich von -78 °C bis $^+$200 °C.

Die gegebenenfalls notwendige Reinigung der erhaltenen Verbindungen erfolgt in üblicher Weise, z.B. durch Säure-Base-Trennung oder chromatographische Verfahren.

Die pharmakologisch akzeptablen Säureadditionssalze erhält man durch an sich bekannte Umsetzung der basischen Verbindungen (I) mit Säuren, welche pharmakologisch verwendbare Salze bilden.

Die folgenden Beispiele 1 bis 3 beschreiben pharmazeutische Zubereitungen enthaltend die erfindungsgemäßen Wirkstoffe sowie die Herstellung solcher pharmazeutischer Zubereitungen.

Beispiel 1 - Tabletten:

| Zusammensetzung: | |
|---|---|
| 17-(3-Methoxybenzyl)-spartein | 20 Teile |
| Maisstärke | 30 Teile |
| Lactose | 55 Teile |
| Kollidon 25® | 5 Teile |
| Magnesiumstearat | 2 Teile |
| Hydriertes Rizinusöl | 1 Teil |
| Total | 113 Teile |

Herstellungsvorschrift:

Der Wirkstoff wird mit Maisstärke und feinpulvriger Lactose in einem Mischer vermischt. Die entstandene Mischung wird mit einer 20 %igen Lösung von Polyvinylpyrrolidon (Kollidon® 25, Fa. BASF) in Isopropanol durchfeuchtet.

Falls erforderlich, wird weiteres Isopropanol hinzugefügt. Das feuchte Granulat wird durch ein 2-mm Sieb passiert, bei 40 °C auf Horden getrocknet und anschließend durch ein 1-mm-Sieb (Frewitt-Maschine) passiert. Nach dem Vermischen des Granulats mit Magnesiumstearat und hydriertem Rizinusöl werden damit Tabletten von 113 mg gepreßt, so daß jede Tablette 20 mg Wirkstoff enthält.

Beispiel 2 - Kapseln

| Zusammensetzung: | |
|---|---|
| 17-(3-Methoxybenzyl)-spartein | 10 Teile |
| Maisstärke | 20 Teile |
| Lactose | 55 Teile |
| Kollidon 25® | 3 Teile |
| Magnesiumstearat | 1,5 Teile |
| Aerosil 200® | 0,5 Teile |
| Total | 90 Teile |

Herstellungsvorschrift:

Der Wirkstoff wird mit Maisstärke und feinpulvriger Lactose in einem Mischer vermischt. Die entstandene Mischung wird mit einer 20 %igen Lösung von Polyvinylpyrrolidon (Kollidon® 25) in Isopropanol durchfeuchtet. Falls erforderlich, wird weiteres Isopropanol hinzugefügt. Das feuchte Granulat wird durch ein 1,6 mm-Sieb (Frewitt) passiert, bei 40 °C auf Horden getrocknet und anschließend durch ein 1 mm-Sieb (Frewitt) passiert. Nach dem Vermischen des Granulats mit Magnesiumstearat und Kieselsäureaerogel (Aerosil® 200, Fa. Degussa) füllt man davon jeweils 90 mg mittels automatischer Kapselmaschine in Hartgelatinekapseln der Größe 4 ab, so daß jede Kapsel 10 mg Wirkstoff enthält.

Beispiel 3 - Ampullen

Zusammensetzung (pro Ampulle):

17-(3-Methoxybenzyl)-spartein 2 mg
Natriumchlorid 16 mg
Wasser für Injektionszwecke ad 2,0 ml

Herstellungsvorschrift:

Man löst Natriumchlorid in Wasser für Injektionszwecke auf, fügt den Wirkstoff hinzu und löst unter Rühren. Mit genügend Wasser für Injektionszwecke wird bis zum Endvolumen aufgefüllt. Der Ansatz wird durch ein Membranfilter 0,25 µm passiert. Man füllt in Braunglasampullen jeweils 2,15 ml ab und schmilzt sie zu. Bei 121 °C wird 30 Minuten lang mit Dampf sterilisiert. 2 ml Injektionslösung enthalten 2 mg Wirkstoff.

Die nachfolgenden Beispiele sollen die Herstellung der Verbindungen der Forml (I) näher erläutern.

Die Strukturen der Verbindungen wurden durch spektroskopische Untersuchungen, insbesondere durch Analyse der NMR-, Massen-, IR und/oder UV-Spektren gesichert.

Das 17-Hydroxyspartein wurde gemäß DE-OS 28 25 117 aus Spartein gewonnen.

Das 17-Dehydrosparteinperchlorat wurde nach M. Rink und K. Grabowski, Arch. Pharm. 289, (1956) 695 aus 17-Hydroxyspartein hergestellt.

In den Beispielen und Tabellen werden folgende Abkürzungen verwendet:

LSM = Lösungsmittel, THF = Tetrahydrofuran, Et = Diethylether, T = Temperatur (°C), Kat = Katalysator, P = 17-Dehydrosparteinperchlorat, H = 17-Hydroxyspartein, DBE = 1,2-Dibromethan, WS = Weinsäure-Salz, HFu = Fumarsäure-Salz.

Beispiel 4: Herstellung der Verbindungen (II)

0,1 mol Magnesiumspäne werden in 50 ml Lösungsmittel (absolutiert) vorgelegt. Gegebenenfalls wird Katalysator zugesetzt. Nach dem Zutropfen von 0,1 mol (IV) in 50 ml Lösungsmittel hält man den Ansatz bis zur Auflösung des Magnesiums am Rückfluß. Der Reaktionsansatz wird als solcher für die Umsetzung in Beispiel 5 verwendet.

Angaben zu speziellen Reaktionsbedingungen für die Herstellung der einzelnen Verbindungen finden sich in der nachfolgenden Tabelle 4.

Beispiel 5: Herstellung von (I) gemäß Variante i)

In den aus Beispiel 4 stammenden Ansatz wird 0,05 mol 17-Hydroxyspartein bzw. 17-Dehydrospartein-Perchlorat in 100 ml Lösungsmittel eingegeben und bis zur vollständigen Umsetzung am Rückfluß erwärmt. Nach vorsichtigem Ansäuern mit verdünnter Salzsäure und anschließender Säure/Base-Trennung und Chromatographie an neutralem Aluminiumoxid bzw. Kieselgel mit Äther/Hexan-Gemisch als Laufmittel wird die Verbindung (I) isoliert. In einer Variante wird diese zu ihrem Säureadditionssalz umgesetzt. Spezielle Reaktionsbedingungen für die Herstellung einzelner Verbindungen und die physikalischen Daten der erhaltenen Produkte werden in Tabelle 5 wiedergegeben.

Beispiel 6: Herstellung von (I) gemäß Variante ii)

6a) Zu 9,2 g 2,3-Dimethoxytoluol in 50 ml absolutem Tetrahydrofuran wurden bei -75 °C 7 ml N,N,N',N'-Tetramemethylethylendiamingegeben. Sodann werden bei dieser Temperatur tropfenweise 37,6 ml einer 1,6-molaren Lösung von n-Butyllithium in Hexan zugegeben. Nach vollendeter Zugabe wird die Temperatur des Reaktionsgemisches langsam auf 0 °C gebracht.

6b) In das aus Beispiel 6a) erhaltene Reaktionsgemisch werden 10 g festes 17-Dehydrospartein-Perchlorat gegeben. Unter langsamer Erwärmung auf Raumtemperatur wird bis zur vollständigen Umsetzung weitergerührt. Anschließend wird die gebildete Verbindung der Formel I wie in Beispiel 5 beschrieben isoliert. Das als farbloses Öl erhaltene 17-(2,3-Dimethoxybenzyl)-spartein-hydrochlorid kann mit etherischer HCl-Lösung in sein amorphes Hydrochlorid überführt werden.

Beispiel 7: Etherspaltung

Zu 0,017 mol 17-(3-Methoxybenzyl)-spartein in 14 ml Essigsäureanhydrid läßt man langsam 30 ml 57 %ige Jodwasserstoff säure tropfen. Der Ansatz wird 4 Stunden unter Rückfluß erhitzt. Man gibt das Reaktionsgemisch vorsichtig auf Eiswasser und führt eine Säure-Base-Trennung durch. Das erhaltene 17-(3-Hydroxybenzyl)-spartein hat einen Schmelzpunkt von 74-76 °C.

<u>Tabelle 4</u> Herstellung von Verbindungen (II) aus (IV)

| Beispiel | LSM | Kat | $R^{1}{}'$, $R^{2}{}'$, $R^{3}{}'$-phenyl | Hal |
|---|---|---|---|---|
| 400 | THF | - | Phenyl | Cl |
| 401 | Et | Jod | 2-Methoxyphenyl | Cl |
| 402 | Et | Jod | 3-Methoxyphenyl | Cl |
| 403 | Et | Jod | 4-Methoxyphenyl | Cl |
| 404 | THF | DBE | 3,4,5-Trimethoxyphenyl | Cl |
| 405 | Et | Jod | 2-Chlorphenyl | Cl |
| 406 | Et | Jod | 3-Chlorphenyl | Cl |
| 407 | Et | - | 4 -Chlorphenyl | Br |
| 408 | Et | - | 3-Trifluormethylphenyl | Cl |
| 409 | Et | - | 2-Fluorphenyl | Br |
| 410 | Et | - | 3-Fluorphenyl | Br |
| 411 | Et | - | 4-Fluorphenyl | Br |
| 412 | Et | - | 2-Bromphenyl | Br |
| 413 | Et | DBE | 3-Bromphenyl | Br |
| 414 | Et | DBE | 4-Bromphenyl | Br |
| 415 | Et | - | 3,4-Dichlorphenyl | Br |
| 416 | Et | - | 2,6-Dichlorphenyl | Br |
| 417 | Et | - | 2-Methylphenyl | Br |
| 418 | Et | - | 3-Methylphenyl | Br |
| 419 | Et | - | 4-Methylphenyl | Br |

<u>Tabelle 4</u> Herstellung von Verbindungen (II) aus (IV) Forts.

| Beispiel | LSM | Kat | $R^{1}$', $R^{2}$', $R^{3}$'-phenyl | Hal |
|---|---|---|---|---|
| 420 | Et | - | 2,4-Dimethylphenyl | Cl |
| 421 | Et | - | 3,5-Dichlorphenyl | Cl |
| 422 | Et | - | 2,6-Difluorphenyl | Br |
| 423 | Et | - | 2-Fluor-3-methylphenyl | Br |
| 424 | Et | - | 2-Chlor-6-fluorphenyl | Cl |

Tabelle 5: Herstellung von Verbindungen (I) gemäß Variante i)

| Beispiel | Ausgangs-Spartein-derivat | LSM | $R^1$, $R^2$, $R^3$-phenyl | Endprodukt isolierte Form | Endprodukt Smp |
|---|---|---|---|---|---|
| 500 | P | THF | Phenyl | Base | 68-71 |
| 501 | P | Et | 2-Methoxyphenyl | Base | 59 |
| 502 | P | Et | 3-Methoxyphenyl | Base | 51-52 |
| 503 | H | Et | 4-Methoxyphenyl | Base | 100 |
| 504 | P | THF | 3,4,5-Trimethoxyphenyl | 2,8 WS | 103 |
| 505 | H | Et | 2-Chlorphenyl | Base | 115 |
| 506 | H | Et | 3-Chlorphenyl | Base | 76 |
| 507 | P | Et | 4-Chlorphenyl | Base | 120 |
| 508 | H | Et | 3-Trifluormethylphenyl | 2,8 HFu | amorph |
| 509 | P | THF | 2-Fluorphenyl | 2,25 WS | 126 |
| 510 | P | THF | 3-Fluorphenyl | 2,15 WS | 138 |
| 511 | P | THF | 4-Fluorphenyl | Base | 74 |
| 512 | P | THF | 2-Bromphenyl | Base | 131 |
| 513 | P | THF | 3-Bromphenyl | 2 WS | 138-140 |
| 514 | P | THF | 4-Bromphenyl | Base | 142 |
| 515 | P | THF | 3,4-Dichlorphenyl | Base | 80 |

Tabelle 5: Herstellung von Verbindungen (I) gemäß Variante i) Forts.

| Beispiel | Ausgangs-Spartein-derivat | LSM | $R^1$, $R^2$, $R^3$-phenyl | Endprodukt isolierte Form | Endprodukt Smp |
|---|---|---|---|---|---|
| 516 | P | THF | 2,6-Dichlorphenyl | 2,1 WS | 143 |
| 517 | P | THF | 2-Methylphenyl | 2,1 WS | 140 |
| 518 | P | THF | 3-Mcthylphenyl | Base | 57 |
| 519 | P | THF | 4-Methylphenyl | Base | 58 |
| 520 | P | THF | 2,4-Dimethylphenyl | Base | 158-161 |
| 521 | P | THF | 3,5-Dichlorphenyl | 2,1 WS | 130 |
| 522 | P | THF | 2,6-Difluorphenyl | 2,2 WS | 135 |
| 523 | P | THF | 2-Fluor-3-methylphenyl | 2,1 WS | 139 |
| 524 | P | THF | 2-Chlor-6-fluorphenyl | 2,2 WS | 131 |

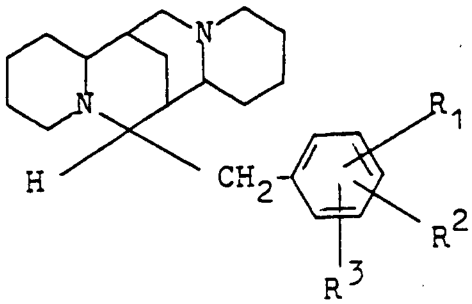

I

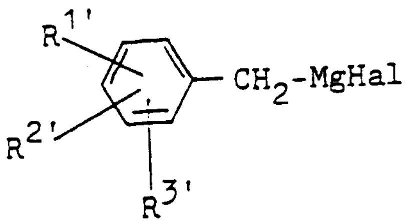

II

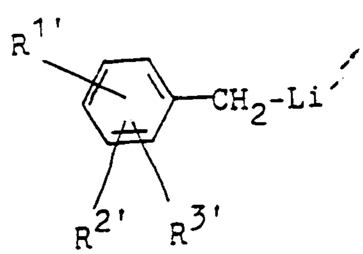

III

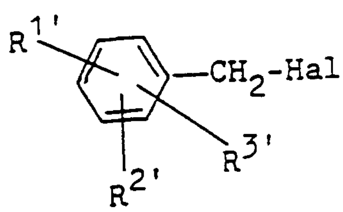

IV

## Patentansprüche

1. Verwendung von 17-Benzylspartein-Derivaten der allgemeinen Formel I

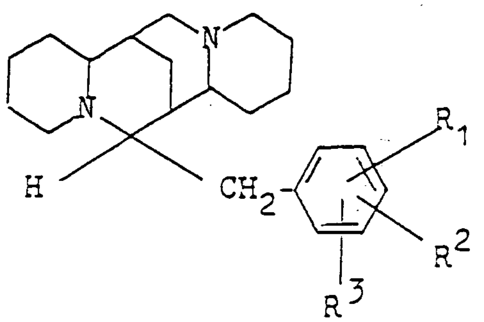

I

worin die Reste $R^1$, $R^2$ und $R^3$ unabhängig voneinander Wasserstoff, Alkyl mit 1-4 Kohlenstoffatomen, Alkoxy mit 1-4 Kohlenstoffatomen, Halogen, Trifluormethyl oder Hydroxy bedeuten und deren physiologisch akzeptablen Säureadditionssalzen zur Herstellung von pharmazeutischen Zubereitungen zur Steigerung der Kontraktionskraft des Herzens in größeren Säugetieren und Menschen.

2. Verwendung von 17-Benzylspartein-Derivaten gemäß Anspruch 1 dadurch gekennzeichnet, daß Verbindungen verwendet werden, worin $R^1$ Wasserstoff, Fluor, Chlor, Hydroxy, Alkoxy mit 1-4 Kohlenstoffatomen oder Alkyl mit 1-4 Kohlenstoffatomen bedeutet, $R^2$ Wasserstoff oder, falls $R^1$ Alkoxy mit 1-4 Kohlenstoffatomen ist, auch Alkoxy mit 1-4 Kohlenstoffatomen bedeutet und $R^3$ Wasserstoff oder, falls $R^2$ und $R^3$ Alkoxy mit 1-4 Kohlenstoffatomen sind, auch Alkoxy mit 1-4 Kohlenstoffatomen bedeutet.

3. Verwendung von 17-Benzylspartein-Derivaten gemäß Anspruch 1, dadurch gekennzeichnet, daß 17-(3-Methoxybenzyl)-spartein oder dessen Säureadditionssalze verwendet werden.

4. Verwendung von 17-Benzylspartein-Derivaten gemäß Anspruch 1, dadurch gekennzeichnet, daß man 17-Benzylspartein-Derivate gemäß Anspruch 1 zusammen mit üblichen pharmazeutischen Hilfsstoffen in eine geeignete Arzneiform überführt.

## Claims

1. The use of 17-benzyl sparteine derivatives of the general formula I,

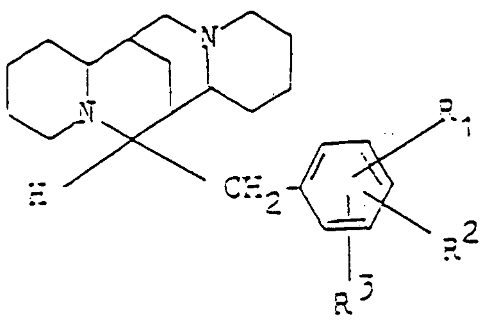

I

wherein the radicals $R^1$, $R^2$ and $R^3$ independently of each other are hydrogen, alkyl with 1-4 carbon atoms, alkoxy with 1-4 carbon atoms, halogen, trifluoromethyl or hydroxy, and their physiologically acceptable acid addition salts for the production of pharmaceutical preparations for increasing the contractivity of the heart in larger mammals and humans.

**2.** The use of 17-benzyl sparteine derivatives according to Claim 1, characterised in that compounds are used in which $R^1$ is hydrogen, fluorine, chlorine, hydroxy, alkoxy with 1-4 carbon atoms or alkyl with 1-4 carbon atoms, $R^2$ is hydrogen or, if $R^1$ is alkoxy with 1-4 carbon atoms, alternatively alkoxy with 1-4 carbon atoms and $R^3$ is hydrogen or, if $R^2$ and $R^3$ are alkoxy with 1-4 carbon atoms, also alkoxy with 1-4 carbon atoms.

**3.** The use of 17-benzyl sparteine derivatives according to Claim 1, characterised in that 17-(3-methoxybenzyl)-sparteine or the acid addition salts thereof is used.

**4.** The use of 17-benzyl sparteine derivatives according to Claim 1, characterised in that 17-benzyl sparteine derivatives according to Claim 1, together with conventional pharmaceutical auxiliaries, are converted into a suitable medicament form.

**Revendications**

**1.** Utilisation de dérivés de 17-benzylspartéine de formule générale I

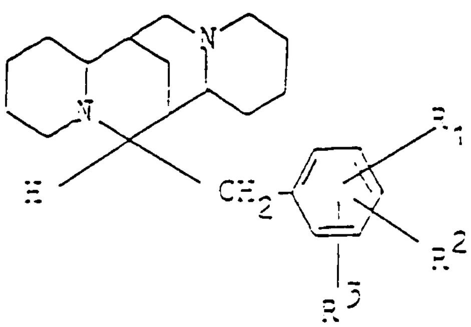

I

dans laquelle les radicaux $R^1$, $R^2$ et $R^3$ représentent, indépendamment les uns des autres, un atome d'hydrogène ou d'halogène ou un groupe alkyle ayant de 1 à 4 atomes de carbone, alcoxy ayant de 1 à 4 atomes de carbone, trifluorométhyle ou hydroxy, et de leurs sels d'addition avec des acides physiologiquement acceptables, pour la préparation de compositions pharmaceutiques destinées à l'augmentation de la contractilité du coeur chez des mammifères supérieurs et chez l'homme.

**2.** Utilisation de dérivés de 17-benzylspartéine selon la revendication 1, caractérisée en ce que l'on utilise des composés dans lesquels $R^1$ représente un atome d'hydrogène, de fluor ou de chlore, ou un groupe hydroxy, alcoxy ayant de 1 à 4 atomes de carbone ou alkyle ayant de 1 à 4 atomes de carbone, $R^2$ représente un atome d'hydrogène, ou, dans le cas où $R^1$ est un groupe alcoxy ayant de 1 à 4 atomes de carbone, également un groupe alcoxy ayant de 1 à 4 atomes de carbone, et $R^3$ représente un atome d'hydrogène ou, dans le cas où $R^2$ et $R^3$ sont des groupes alcoxy ayant de 1 à 4 atomes de carbone, également un groupe alcoxy ayant de 1 à 4 atomes de carbone.

**3.** Utilisation de dérivés de 17-benzylspartéine selon la revendication 1, caractérisée en ce que l'on utilise la 17-(3-méthoxybenzyl)-spartéine ou ses sels d'addition avec des acides.

**4.** Utilisation de dérivés de 17-benzylspartéine selon la revendication 1, caractérisée en ce que l'on transforme en une forme pharmaceutique appropriée des dérivés de 17-benzylspartéine selon la revendication 1, conjointement avec des adjuvants pharmaceutiques usuels.